# EUROPEAN PATENT APPLICATION

(11) **EP 2 474 548 A1**
(43) Date of publication of application: **11.07.2012**
(21) Application number: 10382350.6
(22) Date of filing: 23.12.2010
(51) Int. Cl.: C07D 473/18, C07D 303/14, C07D 303/16, C07C 13/11, C07F 7/08

(54) **Preparation process of an antiviral drug and intermediates thereof**

(71) Applicant: Esteve Química, S.A., 08024 Barcelona (ES)
(72) Inventor: Bartra Sanmartí, Martí, 08024, BARCELONA (ES); Berenguer Maimó, Ramón, 08024, Barcelona (ES); Velasco Turbau, Javier, 08470, Sant Celoni (ES); Ariza Piquer, Javier, 08024, BARCELONA (ES); Farràs Soler, Jaime, 08830, SANT BOI DEL LLOBREGAT (ES); Garcia Gomez, Jorge, 08028 Barcelona (ES)
(74) Representative: ZBM Patents

(57) **Abstract**

Preparation process of an antiviral drug and intermediates thereof
It comprises a preparation process of entecavir comprising: submitting a (1S,3R)-3-(tert-butyldimethylsilyloxy)-1-(oxiran-2-yl)pent-4-yn-1-ol (VIII) to a double esterification and to a radicalary cyclization, yielding a compound of formula (V), where either a compound of formula (VIII) is submitted to a first esterification reaction, then to a catalytic radicalary cyclization using titanocene dichloride as catalyst in the presence of Mn/2,4,6-collidine HCl or Zn/2,4,6-collidine/trimethylsilyl chloride, and finally to a second esterification reaction or, alternatively, the compound of formula (VIII) is submitted first to a catalytic radicalary cyclization, and then to an esterification reaction. Entecavir can be obtained by submitting compound (V) to a desilylation reaction to remove the TBS group and then to a Mitsunobu coupling with 2-amino-6-chloroguanine, followed by hydrolysis. It also relates to some new intermediates of the process.

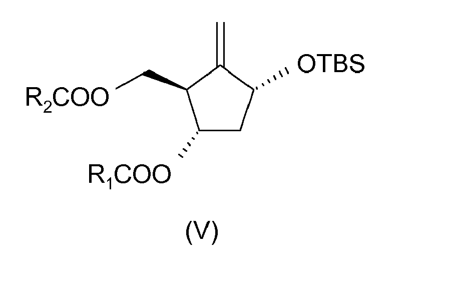

## Description

The present invention relates to a process for the preparation of an antiviral drug known as entecavir, as well as to some new intermediates useful in such preparation process.

### BACKGROUND ART

Entecavir is the International Non-proprietary Name (INN) of 2-amino-9-[(1*S*,3*R*,4*S*)-4-hydroxy-3-(hydroxymethyl)-2-methylidenecyclopentyl]-6,9-dihydro-3*H*-purin-6-one, and CAS No 142217-69-4. Entecavir is marketed as monohydrate under the trade name Baraclude by Bristol-Myers Squibb. It is administered orally in tablet form or as an oral solution containing 0.05 mg/ml. It is a nucleoside analog, more specifically, a guanine analogue, that inhibits reverse transcription, DNA replication and transcription in the viral replication process. Entecavir is used to treat chronic hepatitis B. It also helps to prevent the hepatitis B virus from multiplying and infecting new liver cells. Entecavir is also indicated for the treatment of chronic hepatitis B in adults with HIV/AIDS infection.

The structure of entecavir corresponds to formula (I).

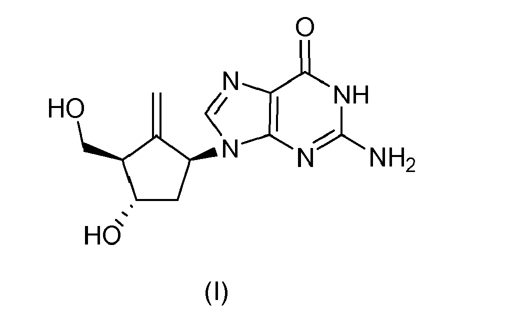

Different synthetic strategies for the preparation of entecavir and its salts are known. The reported processes are mainly focused on how to construct the chiral carbocyclic core and how to introduce the guanidine moiety to the core.

For instance, Example 1 of EP481754-A describes a process for the preparation of entecavir according to the following Scheme 1 comprising the coupling of a protected epoxide with an O-benzyl guanine and subsequent conversion to entecavir. Thus, after introducing the guanine moiety, it is needed a methylenation reaction to complete the construction of the carbocycle moiety.

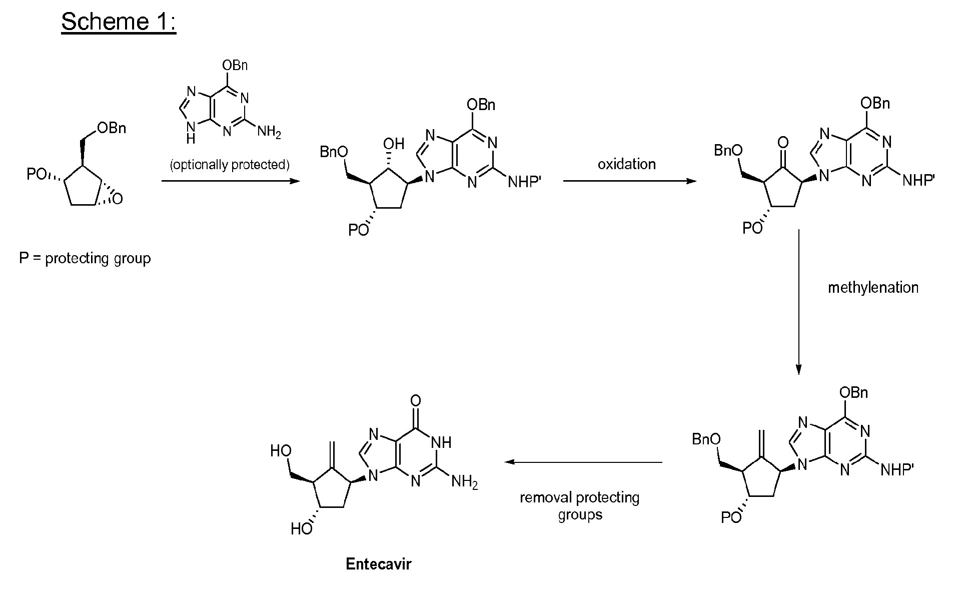

CN1566118-A describes a similar process as the one described above from a protected epoxide where the primary and secondary alcohols are forming an acetal.

Li Wei Guo et al., describe a process for the preparation of entecavir based on the use of Corey lactone diol which is transformed to the compound (1) below. This compound is reacted with O-benzyl guanine and subsequently transformed into entecavir (cf. Li Wei Guo et al., "A new route for synthesis of Entecavir", Chinese Chemical Letters 2006 vol. 17, pp. 907-910).

CN1861602-A describes a process for the preparation of entecavir also based on the use of the compound (1). 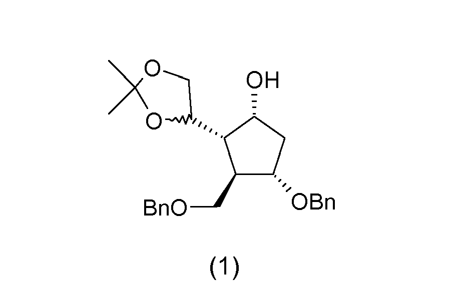 W02004/52310-A describes several processes for the preparation of entecavir. Some of them are based on the coupling via a cyclopentane epoxide or a cyclopentanol with a substituted guanine precursor to yield a carbocyclic nucleoside intermediate, followed by subsequent conversion of the compound obtained into entecavir. Another process is based on the coupling of a substituted methylene cyclopentyl amine with a substituted chloropyrimidine followed by conversion first to a purine derivative and then to entecavir.

Other known processes are focused on the use of an intermediate olefin cyclopentane of formula (2) below, where R₁, R₂ are protective groups and R₃ is H or a protective group. The cyclopentyl olefin is then submitted to a Mitsunobu reaction with a guanine precursor to yield a carbocyclic nucleoside intermediate, followed by removal of the protective groups and hydrolysis to yield entecavir. 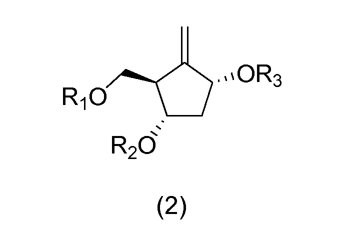 Thus, the W02004/52310-A mentioned above also describes processes for the preparation of entecavir comprising the use of an intermediate olefin compound as the one of formula (2) where R₃ is H, and R₁ and R₂ are equal protective groups selected from silyl or carbonyl groups. The preparation of the compound with silyl groups is carried out starting from a bicyclic lactone namely as Corey lactone diol which is submitted to several reaction steps including a reduction, iodination by free radical oxidation, and olefination reactions. The preparation of the compound with a carbonyl group is summarized in Scheme 2.

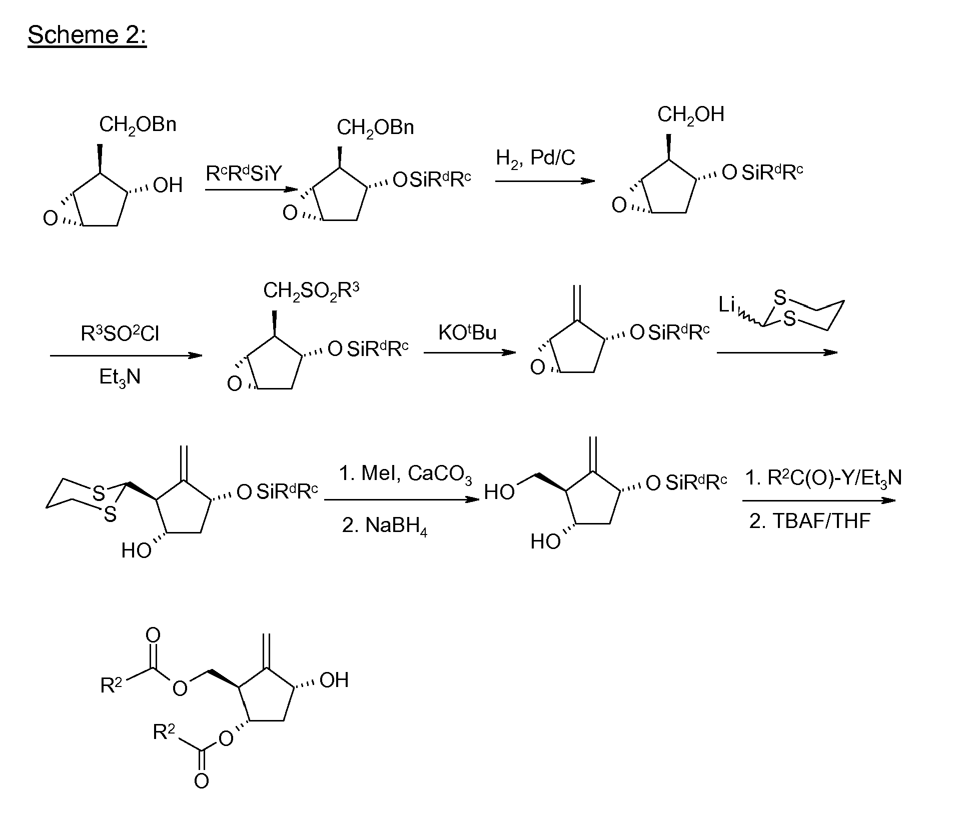

WO2010/74534-A describes a process for the preparation of entecavir based on carrying out an olefination of an α-hydroxy ketone compound according to the following Scheme 3 with a mixture of Nysted reagent and TiCl₄, a Wittig reagent or a Tebbe reagent to give a compound of formula (2) where R₁ and R₂ are independently H or an OH protective group, or both are forming part of a cyclic OH protective group, and R₃ is H, alkylsilyl, or allylsilyl.

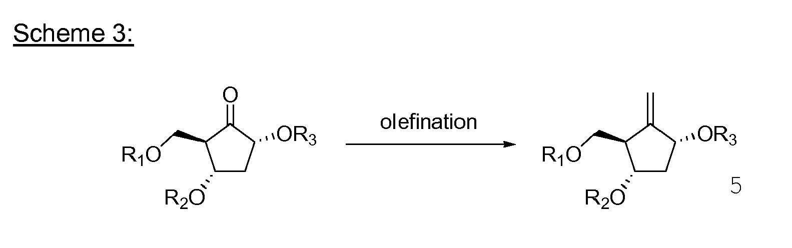

According to the Example 3 of page 17 it is prepared a compound (2) where R₁ is CPh₃, R₂ is tert-butyldiphenylsilyl (TBDPS) and R₃ = H.

Most of the processes summarized above use reagents which are not appropriate at industrial scale, for instance, in WO 2004/52310 methyl iodide is used which is a potential cancerigen compound. In WO2010/74534 the Nysted reagent is used which is a dangerous reagent that reacts violently with water and forms explosive peroxides. Likewise, the cyclopentadiene used as starting material in Example 1 of EP481754 is not stable and dimerizes easily. Therefore, it has to be carefully manipulated at low temperatures,

It has been also described in the art the preparation of a compound of formula (2) by radical cyclization. Thus, F.E: Ziegler et al., describes a process for the preparation of a compound of formula (2) where R₁ is pivaloyl, R₂ is tert-butyldimethylsilyl (TBS) and R₃ is H which is useful for the preparation of entecavir via Mitsunobu coupling with guanine. This compound is prepared via bis-silylation of diol (3) thereby both hydroxyl groups are protected with TBS groups, and subsequent epoxidation, followed by radical cyclization using titanocene dichloride (Cp₂TiCl₂), protection of the free primary hydroxyl group of the cyclic compound as a pivaloyl ester (R₁), and selective desilylation of the secondary allylic silyl ether (R₃) (cf. F.E: Ziegler et al. "Radical cyclization studies directed toward the synthesis of BMS-200475 'entecavir': the carbocyclic core", Tetrahedron 2003, vol. 59, pp. 9013-9018). 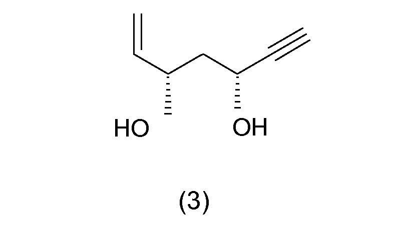 Unfortunately, the radical cyclization needs even an amount higher than the stoichiometric amount of titanocene dichloride which renders the process not industrializable. The use of a large amount of catalyst gives rise to problems in the work up and to need a huge amount of solvent to avoid a sub product of an elimination reaction. Besides, the desilylation to remove the TBS group of the secondary allylic ether is not selective yielding to low yields of the desired compound.

The previous document also describes a process for preparing the diol (3) from a D-diacetone glucose compound according to the following Scheme 4:

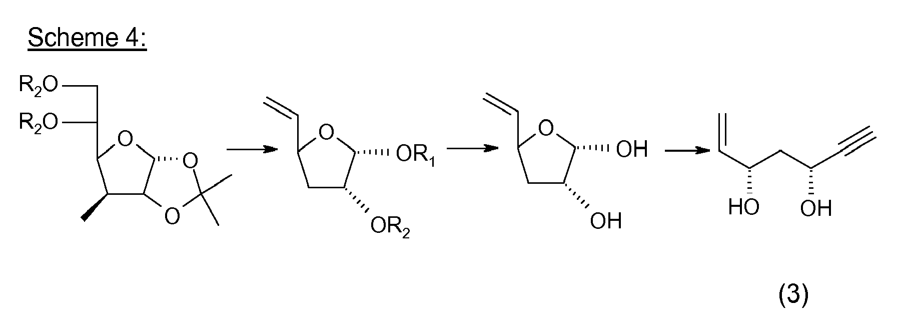

The diol (3) is an oil which make difficult its purification. Besides, this process for preparing diol (3) uses reagents which are not appropriate at industrial scale such as carbon disulfide which is mutagenic, methyl iodide which is potentially cancerigen, and (Bu₃Sn)₂O which is highly toxic.

Finally, a syn selective reduction of β-hydroxy acetylenic ketones to yield syn-1,3-diols is also known in the art. The syn-1,3-diols obtained is a racemic mixture of the two syn-isomers. Thus, the diol (3) is prepared by direct reduction of the corresponding β-hydroxy acetylenic ketone with diisobutylaluminium hydride (DIBAL-H) (cf. J.A. Funel et al., "Enyne versus diene RCM in the synthesis of cyclopentene derivatives toward the A ring of FR181877", J.Org. Chem. 2004, vol. 69, pp. 4555-4558). Likewise, the selective reduction with DIBAL-H has also been described for the preparation of diol (3) with a TMS in the alkyne position, compound (±)(4), as it is illustrated in the Scheme 5 below (cf. Peter Mohr, "Stereoselective synthesis of syn-1,3-diols", Tetrahedron letters 1991, vol. 32, pp. 2219-2229). However, although the reduction is selective syn vs. trans, it cannot afford a unique stereoisomer but a racemic mixture of the two syn-1-3diols. 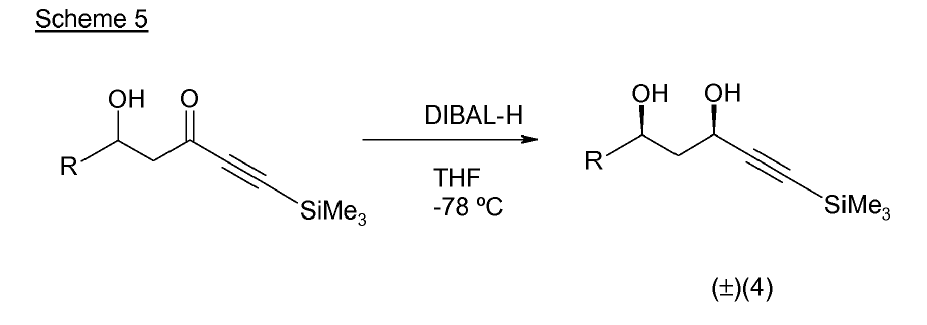 Despite the teaching of all these prior art documents, the research of new preparation processes of entecavir is still an active field, since the industrial exploitation of known processes is difficult. Thus, the provision of an efficient and amenable to scale-up preparation process of entecavir is desirable.

### SUMMARY OF THE INVENTION

Inventors have found a process for the preparation of entecavir comprising the construction of the chiral carbocyclic core by radical cyclization of a linear precursor having the hydroxyl groups differentiated, by orthogonally protection with a tert-butyldimethylsilyl (TBS) ether and an ester and which works with a catalytic amount of catalyst, overcoming the disadvantages mentioned above by the known process that goes through a radical cyclization described by F.E: Ziegler et al., in Tetrahedron 2003, vol. 59, pp. 9013-9018. Therefore, high dilution reaction conditions are avoided, the amount of metal to be used is reduced, the reaction time is also reduced, the process and the work-up are simpler, it is also more economic and it is industrializable. Besides, it is considerably diminished the formation of a sub product due to a reductive elimination secondary reaction.

Besides, a subsequent selective desilylation to remove the TBS group of the secondary allylic alcohol can be carried out yielding good yields of the desired compound. It has been possible thanks to the inventors finding of different protective groups which have resulted to be specially suitable in the protection/deprotection reactions of the secondary alcohols forming part of the olefin cyclopentane moiety of the entecavir and in the radical cyclization reaction, as well as the finding of specific selective protection/deprotection conditions of each secondary alcohol which allow to carry out the reactions in a selective manner.

Accordingly, an aspect of the present invention is the provision of a process for the preparation of entecavir of formula (I), or its pharmaceutically acceptable salts, 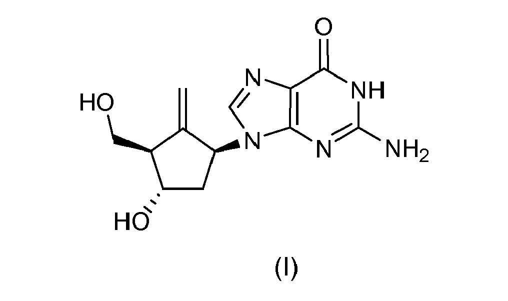 which comprises: submitting a compound of formula (VIII) to a double esterification and to a catalytic radicalary cyclization using titanocene dichloride as catalyst in the presence of Mn/2,4,6-collidine HCl or Zn/2,4,6-collidine/trimethylsilyl chloride, yielding to a compound of formula (V), 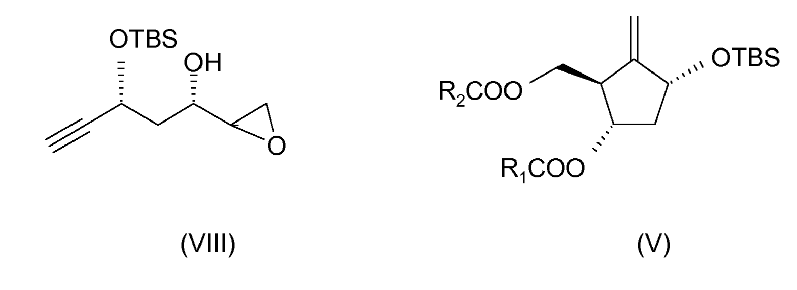 wherein: either a compound of formula (VIII) is submitted to a first esterification reaction, yielding to a compound of formula (VII); 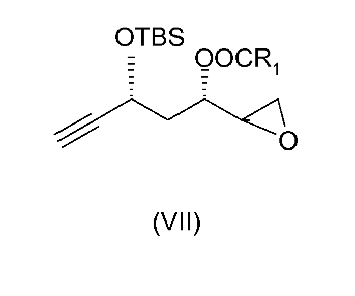 then to the catalytic radicalary cyclization to yield a compound of formula (VIa); followed by a second esterification reaction, to yield a compound of formula (V); 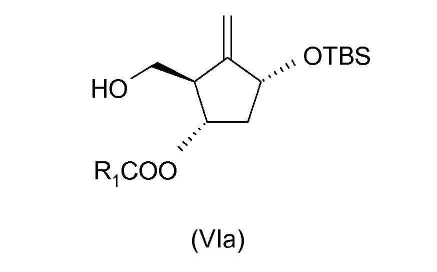 or, alternatively, the compound of formula (VIII) is submitted first to the catalytic radicalary cyclization to yield a compound of formula (VIb); and then to a esterification reaction, to yield a compound of formula (V); and 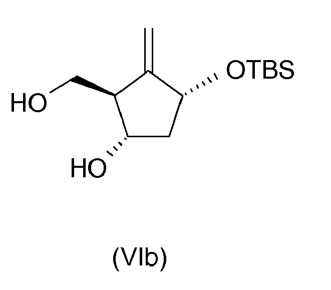 wherein: R₁ and R₂ are radicals independently selected from the group consisting of (C₁-C₆)-alkyl, phenyl, and phenyl substituted by a radical selected from the group consisting of (C₁-C₄)-alkyl, (C₁-C₄)-alkoxy, halogen, and nitro.

Compound of formula (VIII) can be prepared by epoxidating a compound of formula (IXa) using an epoxidating agent in a suitable solvent.

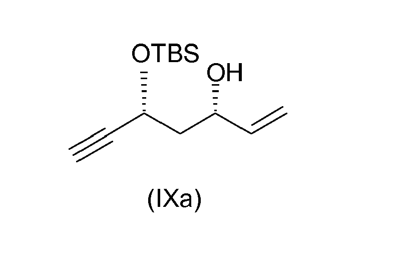

Compound of formula (IXa) can be prepared by submitting a compound of formula (X) to a desilylation reaction.

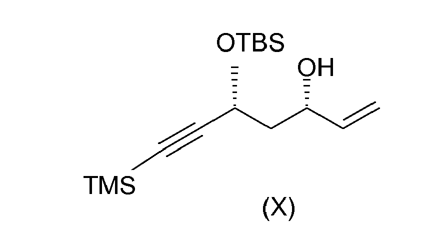

Alternatively, compound of formula (VIII) can be prepared by submitting a compound of formula (IXb) to a desilylation reaction.

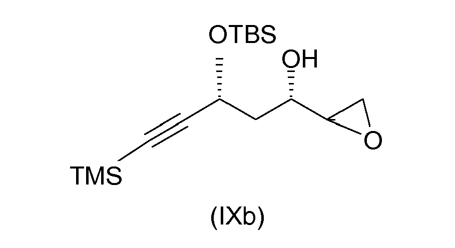

Compound of formula (IXb) can be prepared by epoxidating a compound of formula (X) using an epoxidating agent in a suitable solvent.

Compound of formula (X) can be prepared by reacting a compound of formula (XI) with tert-butyldimethylsilyl chloride, in the presence of imidazole and a suitable solvent.

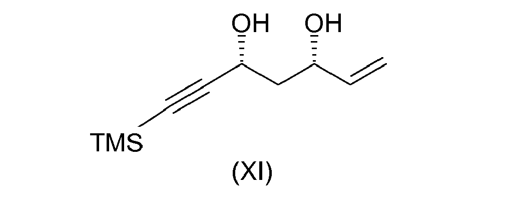

Compound of formula (XI) is the isomer (3S,5R)-7-(trimethylsilyl)hept-1-en-6-yne-3,5-diol. This specific isomer can be prepared by an aldol reaction via boro enolate between 4-trimethylsilyl-3-butyn-2-one of formula (XII) which is reacted with (+)-B-chlorodiisopinocampheylborane ((+)-DIPCI) in the presence of an organic tertiary amine and a suitable solvent and then with a compound of formula (XIII), followed by submitting the compound thus obtained to a syn stereoselective reduction using a reducing agent, and to a subsequent treatment to cleavage the boron moiety.

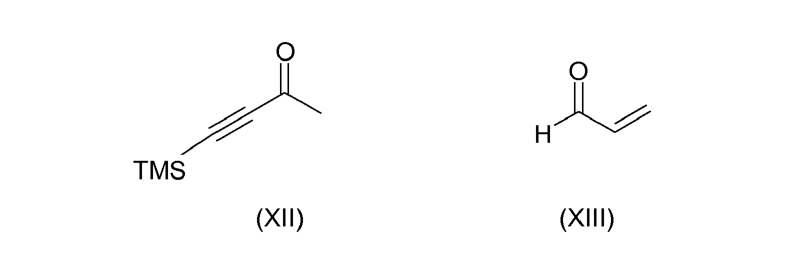

Entecavir can be prepared from the key intermediate of formula (V) by a process comprising a selective desilylation reaction to remove the TBS group, a Mitsunobu reaction between the compound obtained with a 6-halo-2-aminopurine, in particular with 6-chloro-2-aminopurine, a hydrolysis reaction to remove the esters groups, and if desired reacting the entecavir obtained with a pharmaceutically acceptable acid or with a pharmaceutically acceptable base to yield a pharmaceutically acceptable salt.

Several steps of the global process represents by their own a significant improvement over the known prior art. Additionally, when different steps are carried out together the resulting process is particularly effective at industrial scale. Thus, it is also part of the present invention the provision of single reaction steps of the process comprising the sequence of steps described above from the starting compound of formula (XII) to the preparation of the intermediate compound of formula (V), as well as the combination of two or more sequential steps of this process.

Several intermediate compounds are new and form part of the invention. Thus, another aspect of the present invention is the provision of the compounds of formula (XI), (X), (IX), (VIII), (VII), and (VIa).

### DETAILED DESCRIPTION OF THE INVENTION

All terms as used herein in this application, unless otherwise stated, shall be understood in their ordinary meaning as known in the art. Other more specific definitions for certain terms as used in the present application are as set forth below and are intended to apply uniformly through-out the specification and claims unless an otherwise expressly set out definition provides a broader definition.

In the formula of the compounds of the present invention, the use of bold and dashed lines to denote particular configuration of groups follows the IUPAC convention. A bond indicated by a broken line indicates that the group in question is below the general plane of the molecule as drawn (the "alpha" configuration), and a bond indicated by a bold line indicates that the group at the position in question is above the general plane of the molecule as drawn (the "beta" configuration).

The term "pharmaceutically acceptable salts" refers to salts prepared from pharmaceutically acceptable non-toxic bases or acids, including inorganic or organic bases and inorganic or organic acids. As the entecavir is a basic compound, salts may be prepared form pharmaceutically acceptable non-toxic acids, including inorganic and organic acids. Such acids include for instance acetic, benzenesulfonic, benzoic, camphorsulfonic, citric, ethansulfonic, fumaric, gluconic, glutamic, hydrobromic, hydrochloric, lactic, maleic, malic, mandelic, methanesulfonic, phosphoric, succinic, sulfuric, tartaric, p-toluensulfonic acid, and the like. Particularly preferred are citric, hydrobromic, hydrochloric, maleic, sulfuric, fumaric, and tartaric acids.

It will be understood that, as used herein, references to entecavir are meant to also include the pharmaceutically acceptable salts.

In the context of the invention, the term "selective reaction" refers to one that can occur with other substances or with different functional groups of the same substance but exhibits a degree of preference for the substance of interest or for the functional group of interest.

In the context of the invention, the term "stereoselective" refers to a given reaction that yields more of one stereoisomer of a set of stereoisomers.

As mentioned above, it is provided a process for the preparation of entecavir which comprises several steps encompassing the transformation of a compound of formula (VIII) to a compound of formula (V). Subsequently, compound (V) can then be transformed into entecavir.

Additionally, compound of formula (VIII) can be prepared from commercial starting materials, compound (XII) and compound (XIII), by a sequence of reactions which contribute to obtain a chiral carbocyclic core having the hydroxyl groups differentiated, preferably by orthogonally protection with a TBS and an ester. More preferably, the ester is an acetyl (Ac) ester. This sequence of reactions includes the preparation of a specific steroisomer of the compound of formula (XI) by a highly stereoselective process which affords the desired stereoisomer of the free diol among the four possible stereoisomers. The selective monoprotection of the hydroxy of the propargylic position is achieved by the specific selection of the protective group TBS and the reaction conditions used. It also comprises a subsequent desilylation and epoxidation which can be carried out in any order.

The following Scheme I illustrates a particular embodiment of the global process starting from commercial compounds and for the case that the protective groups are TBS and acetyl. 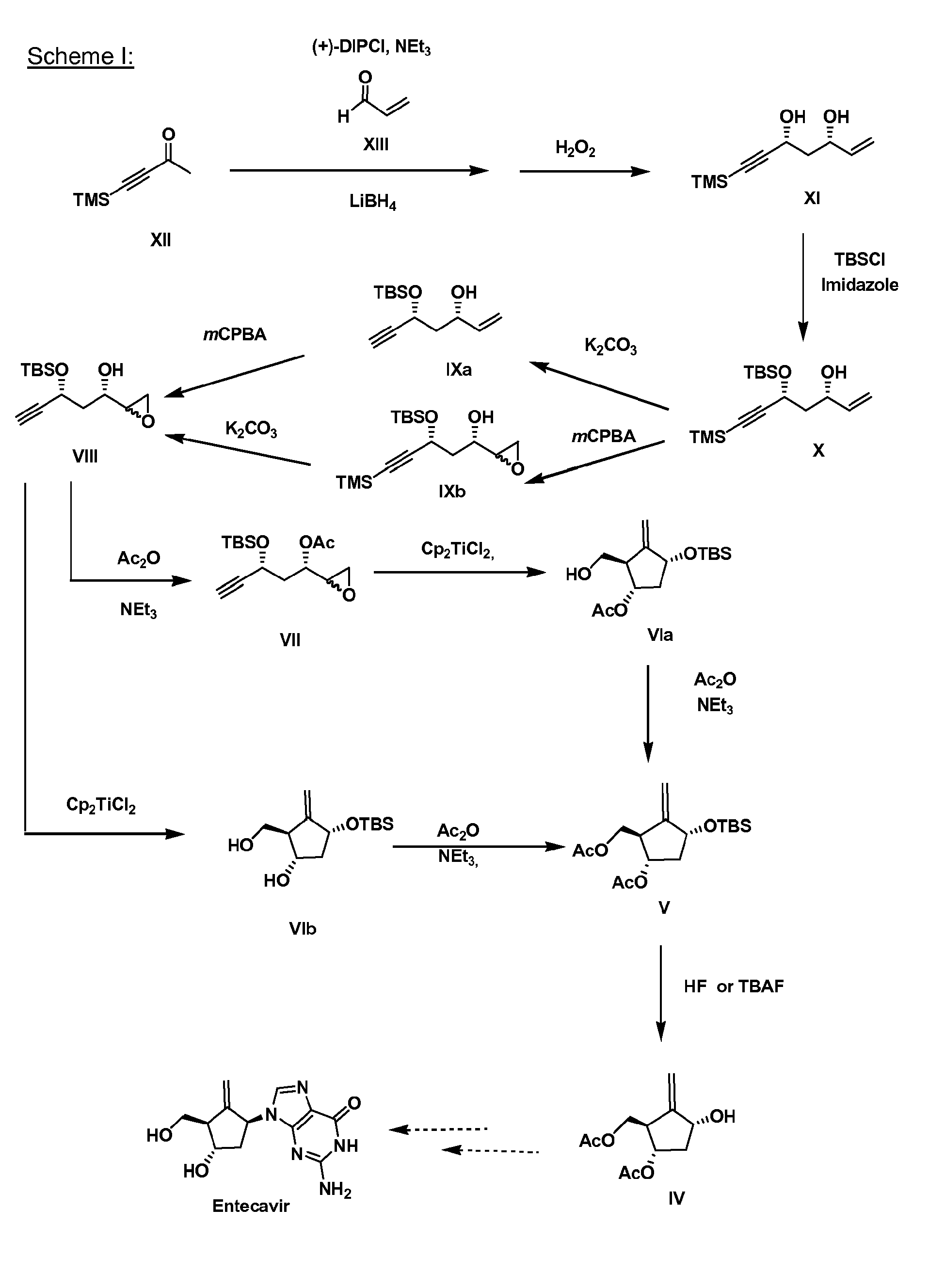 Hereinafter, a description of the preparation process by each step will be given in detail.

Compound of formula (XI) is the following syn isomer: (3S,5R)-7-(trimethylsilyl)hept-1-en-6-yne-3,5-diol. It is a new intermediate useful in the preparation process of entecavir of the present invention. The preparation process of the compound of formula (XI) is new and also forms part of the invention. The process is highly stereoselective.

It can be prepared from the compound of formula (XII) and acrolein (XIII), both commercially available.

Thus, 4-trimethylsilyl-3-butyn-2-one of formula (XII) can be reacted first with (+)-DIPCI and a tertiary amine in a suitable solvent, and then with acrolein of formula (XIII), followed by submitting the compound thus obtained to a syn stereoselective reduction using a reducing agent such as lithium borohydride or sodium borohydride. The boron compound intermediate thus obtained is then treated with hydrogen peroxide, methanolamine, ethanolamine, or triethanolamine to cleavage the boron moiety yielding to the compound of formula (XI). Preferably, this sequence of steps are carried out in one pot, thereby the process is more efficient and avoids the isolation of unstable intermediates.

Preferably, the tertiary amine is selected from the group consisting of: triethylamine, tributylamine, tripropylamine, N,N-diisopropylethylamine, and N,N,N',N'-tetramethylethane 1,2-diamine. More preferably, the tertiary amine is triethylamine.

Preferably, the suitable solvent used in the reaction between compound (XII) and compound (XIII) is selected from a (C₃-C₆)-ether such as tetrahydrofuran, (C₁-C₆) chlorine containing solvents such as chloroform or dichloromethane, and their mixtures with (C₅-C₇) alkanes such as pentane, hexane or cyclohexane More preferably, the ether is tetrahydrofuran. The reaction is generally carried out at low temperatures, generally at -78 °C. Once the reaction is considered completed, it can be quenched for instance with a saturated solution of ammonium chloride.

Preferably, the final treatment of the boron compound intermediate is carried out with H₂O₂. Preferably, the solvent is selected from a mixture of a (C₃-C₆)-ether and water, (C₁-C₄)-alcohol, and their mixtures with (C₅-C₇) alkanes such as pentane, hexane or cyclohexane.

The compound of formula (XI) is solid, which is advantageous since its manipulation and purification is easier to carry out, especially if an enrichment of the optical purity is desired.

Compound of formula (XI) can be monoprotected with a high selectivity in the propargylic position by reacting it with TBSCI in the presence of imidazole and a suitable solvent to afford the compound of formula (X).

The selection of the specific protective group TBS to carry out the selective monoprotection of the compound of formula (XI) is a key feature of this step of the process. Other protective groups such as the tert-butyldiphenylsilyl (TBDPS) or benzoyl (Bz) give rise to a low selectivity yielding mixtures of monoprotection/ diprotection and low yields. The triisopropylsilyl (TIPS) gives quite good selectivities, but the following radical cyclization with the compound having this protective group does not work. The presence of imidazole as base also contributes to the selectivity of the reaction. Other weak bases as pyridine or strong bases such as diisopropylethylamine or triethylamine give rise to a low selectivity mono vs. diprotection and also to a mixture of monoprotected compounds as it is illustrated in the Examples.

The monoprotection reaction is carried out in the presence of a suitable solvent which is selected from the group consisting of (C₃-C₆)-ethers and (C₁-C₇)-chlorine containing solvents. Preferably, the solvent is selected from tetrahydrofuran and dichloromethane.

In a preferred embodiment, the subsequent sequence of reactions comprises desilylation, epoxidation, and protection of the secondary hydroxyl as an ester to yield the compound of formula (VII). Preferably, the hydroxyl protective group is an acetyl. This sequence of steps yields to an oxiran pentynyl intermediate having two hydroxy groups orthogonally protected with different protective groups (TBS ether and ester). The process is easy to carry out yielding compounds that are easy to purify. Thus, good yields and purity are obtained using this sequence of reaction steps.

The desilylation reaction of the compound of formula (X) can be carried out using an inorganic base such as an alkaline metal carbonate or a alkaline hydroxide such as sodium hydroxide or potassium hydroxide, or diaza(1,3)bicyclo[5.4.0]undecane (DBU), in the presence of a solvent selected from (C₁-C₄)-alcohol, acetonitrile and mixtures of any of them with water. Preferably, the alkaline metal carbonate is sodium carbonate or potassium carbonate. More preferably, the alkaline metal carbonate is potassium carbonate. Also preferably, the alcohol is methanol.

The epoxidation reaction of the compound of formula (IXa) can be carried out with an epoxidating agent in a suitable solvent. Preferably, the epoxidating agent is selected from the group consisting of m-chloroperbenzoic acid (m-CPBA), t-butyl hydroperoxide and cumenyl hydroperoxide. More preferably, the epoxidating agent is m-CPBA. The epoxidation can be carried out in the presence of a catalyst.. The catalyst can be a metal compound of vanadium, titanium or molybdene. Preferably the catalyst is vanadyl acetylacetonate. Examples of suitable solvents are (C₁-C₆)-chlorine containing solvents such as dichloromethane. Generally, this reaction is carried out at room temperature.

The conversion of the compound of formula (X) to the compound of formula (VIII) can also be carried out submitting the compound (X) first to the epoxidation reaction and then to the desilylation reaction in the same conditions as described above.

The esterification of the hydroxyl group of the compound of formula (VIII) previously obtained to yield the compound of formula (VII) can be carried out using a (C₄-C₁₀)-anhydride or an (C₂-C₆)-acyl halide in the presence of a suitable solvent and a base. Examples of suitable solvents include (C₁-C₆)-chlorine containing solvents such as dichloromethane, (C₂-C₆)-ethers, (C₆-C₈)-aromatic hydrocarbons such as toluene, pyridine, or N,N-dimethylformamide. Preferably, the base is a tertiary amine. More preferably, the tertiary amine is triethylamine, tributylamine or tripropylamine. Even more preferably, the base is triethylamine or the system triethylamine/4-dimethylaminopyridine, the 4-dimethylaminopyridine being in a catalytic amount. Other organic amines can also be used such a diisopropylethylamine, pyridine, 4-dimethylaminopyridine, collidine or imidazole.

In a preferred embodiment, the esterification is an acetylation. Examples of acetylating agents are acetic anhydride or acetyl chloride. Preferably, the acetylating agent is acetic anhydride.

In a preferred embodiment of the process the compound of formula (VII) is submitted to a catalytic radicalary cyclization reaction.

As mentioned above, it is also a key feature of the process the expedient and highly stereoselective catalytic radical cyclization reaction which can be carried out from an orthogonally protected compound of formula (VII) Complete selectivity is achieved, allowing for the preparation of the new five-membered pentane compound of formula (VIa) with high stereoselectivity.

Inventors have found that the hydroxyl of the propargyl position must be protected as TBS. With other protective groups the cyclization does not work e.g. TIPS protective group, or it works but without diastereoselectivity, e.g. benzoyl protective group. The other hydroxyl group is protected as an ester, preferably as an acetyl ester. This specific combination of protective groups allows carrying out the cyclization with quite good yields and stereoselectivity.

The radical cyclization of the present invention works with catalytic amounts of catalyst which is advantageous since high dilution reaction conditions are avoided, the amount of catalyst to be used is considerably reduced, the reaction time is also reduced, the purification is easier, and the preparation process is simpler and more economic. Besides, the reductive elimination, which is a secondary reaction, is minimized.

By the term "catalytic radicalary cyclization" is understood a radical cyclization that works with an amount of catalyst lower than the stochiometric amount.

Generally, the amount of Cp₂TiCl₂ is comprised between 10-30% w/w in respect of the compound of formula (VII) or in respect of the compound of formula (VIII). Preferably, the amount of catalyst is 20% w/w.

In a preferred embodiment a mixture of Zn, 2,4,6-collidine and TMSCI is used to recover the catalyst. Preferably, the ratio molar of Zn in respect of the starting compound of formula (VII) is 3 equivalents per equivalent of starting compound. The equivalents of 2,4,6-collidine are generally comprised between 5-8 equivalents per equivalent of starting compound and the equivalents of TMSCI are generally comprised between 2.5 and 4 equivalents per equivalent of the starting compound. Preferably, the equivalents of 2,4,6-collidine are comprised between 5-6 equivalents per equivalent of the starting compound. Also preferably, the equivalents of TMSCI are comprised between 2.5-3 equivalents per equivalent of starting compound.

In a particular embodiment, a mixture of Mn and 2,4,6-collidine hydrochloride is used to carry out the cyclization of the compound of formula (VII). Preferably, the ratio molar of Mn in respect of the starting compound of formula (VII) is 3 equivalents per equivalent of starting compound and the equivalents of 2,4,6-collidine·HCl are generally comprised between 2.5-4 equivalents per equivalent of starting compound.

Generally the solvent used is an (C₃-C₆)-ether such as tetrahydrofuran or 2-methyl tetrahydrofuran.

In another embodiment of the process compound of formula (VIII) is directly submitted to the cyclization reaction in the conditions described above, to yield a compound of formula (VIb).

Compound (VIa) or compound (VIb) obtained after the radicalary cyclization are submitted to an esterification reaction in the same conditions as mentioned above in order to protect the free OH groups as ester groups. Compound (V) thus obtained has the two secondary hydroxyl groups protected with two different protective groups. Thus, a selective desilylation of the hydroxyl group having the TBS protective group can be achieved in good yields and selectivity.

Preferably, the esterification is an acetylation reaction. The acetylation of the free hydroxyl groups as acetyl groups can be carried out using an acetylating agent in the presence of a suitable solvent and a organic base. Preferred bases, solvents, and acetylating agents are the ones described above for the esterification of the compound of formula (VIII).

In a preferred embodiment, the catalytic radicalary cyclization and the subsequent acetylation are carried out in one pot, without isolation of the compound of formula (VIa) or (VIb).

The compound of formula (V) is a key intermediate in the preparation of entecavir. Entecavir can be prepared from this compound as it is described in Example 14 of WO2004052310.

In a particular embodiment of the process, entecavir is prepared from the compound of formula (V) by a process comprising first submitting the compound of formula (V) to a desilylation reaction to remove the TBS group using, for instance, fluorhidric acid or tetra-n-butyl ammonium fluoride in a suitable solvent, yielding to a compound of formula (IV), 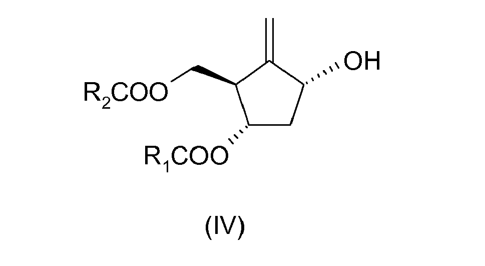 then, carrying out a Mitsunobu reaction between the compound of formula (IV) and a compound of formula (III) to yield a compound of formula (II), 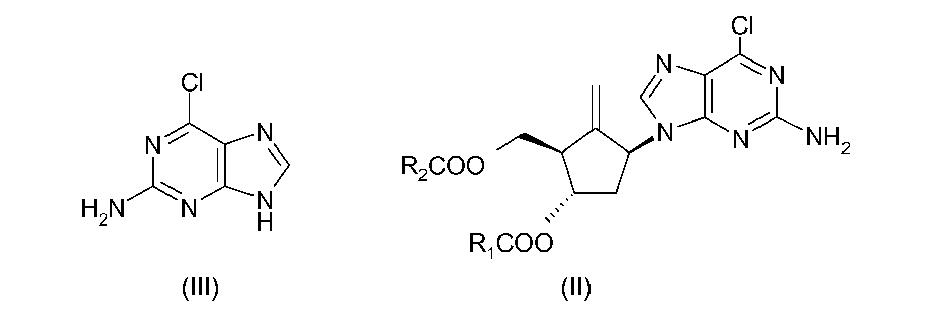 followed by submitting the compound of formula (II) to a hydrolysis reaction yielding entecavir, and if desired reacting the entecavir obtained with a pharmaceutically acceptable acid to yield a pharmaceutically acceptable salt.

In formula (IV) and in formula (II), R₁ and R₂ are radicals independently selected from the group consisting of (C₁-C₆)-alkyl, phenyl, and phenyl substituted by a radical selected from the group consisting of (C₁-C₄)-alkyl, (C₁-C₄)-alkoxy, halogen, and nitro.

The selective desilylation to remove the TBS group can be carried out with fluorhidric acid, tetra-n-butyl ammonium fluoride (TBAF), (±) β-camphorsulfonic acid, or p-toluensulfonic acid (TsOH). Generally, the solvent used is selected from tetrahydrofuran, pyridine, and a mixture thereof. Generally, the reaction is carried out at room temperature.

The use of compound of formula (III) is preferred to other halopyrimidines to carry out the Mitsunobu coupling. The reaction proceeds with good yields and purity. Besides, the cloropyrimidine is preferred at industrial scale. The Mitsunobu reaction is carried out in the presence of a phosphine and an azodicarboxylate compound. The phosphine can be triphenylphosphine or (C₁-C₄)-trialkylphosphine such as tributylphosphine. The azodicarboxylate can be diethylazodicarboxylate (DEAD) or diisopropylazodicarboxylate (DIAD). Generally the solvents used are tetrahydrofuran, N,N-dimethylformamide or mixtures thereof. Other solvents such as (C₆-C₈) aromatic hydrocarbons such as toluene or (C₁-C₄)-alkyl-(C₂-C₆)-alkanoates such as ethyl acetate can also be used. Generally, the reaction is carried out at a temperature comprised between room temperature (22 °C) and -40 °C. Preferably, at a temperature comprised between -10°C and -40 °C.

Finally, the hydrolysis of the compound obtained can be carried out by known processes, for instance as the one described in W02004/52310. Thus, the hydrolysis can be carried out using an inorganic base in a suitable solvent. Examples of suitable inorganic bases are metal alkaline hydroxide such sodium or potassium hydroxide.

The most adequate conditions for carrying out the process vary depending on the parameters considered by an expert in the art, such as the concentration, the temperature, the solvent used during the reaction or the isolation of the products, and the like. These can be readily determined by said skilled person in the art with the help of the teachings of the examples given in this description.

The entecavir can be isolated as a pharmaceutically acceptable salt. Several salts of entecavir are known in the art. A salt can be obtained from the entecavir base by treatment with the corresponding acid. It can also be obtained by treatment with the corresponding base. Alternatively, a salt of the entecavir obtainable according to the process of the invention is converted into another salt.

The entecavir, including their salts can exist in solvated, as well as unsolvated forms, including hydrated forms. Thus, they can contain in its structure stoichiometric amounts of solvent in the case of solvates, or of water in the case of hydrates. It is to be understood that the process of the invention encompasses the preparation of all such solvated, as well as unsolvated forms. The obtention of solvates and hydrates depends on the solvent used and the crystallization conditions that can be determined by the skilled person.

As mentioned above several intermediates compounds of this preparation process are new and form part of the invention. Thus, compounds of formula (XI), (X), (IX), (VIII), (VII), and (VIa) are also part of the invention.

In a preferred embodiment, the compound of formula (VII) is that where R₁ is methyl.

In another preferred embodiment, the compound of formula (VIa) is that where R₁ is methyl.

Throughout the description and claims the word "comprise" and variations of the word, are not intended to exclude other technical features, additives, components, or steps. Additional objects, advantages and features of the invention will become apparent to those skilled in the art upon examination of the description or may be learned by practice of the invention. The following examples are provided by way of illustration, and they are not intended to be limiting of the present invention. Furthermore, the present invention covers all possible combinations of particular and preferred embodiments described herein.

### EXAMPLES

### Example 1: Preparation of (3S,5R)-7-(trimethylsilyl)hept-1-en-6-yne-3,5-diol (XI)

To a solution of (+)-DIPCI (90-105 %) (25 g, 77.94 mmol) in anhydrous tetrahydrofuran (THF) (40 mL) at 0 °C under N₂ atmosphere, triethylamine (NEt₃) (85.02 mmol, 11.6 mL) was added under stirring. Then, 4-trimethylsilyl-3-butyn-2-one 98% (70.85 mmol, 9.78 g) was added dropwise and the reaction mixture was stirred 2 h at - 5 °C - 0 °C. Then the solution was cooled to - 78 °C. A solution of acrolein (90% purity) (102.6 mmol, 7.62 mL) in anhydrous THF (20 mL) was added slowly and the reaction mixture was stirred 1 h at - 78 °C. A solution of lithium borohydride (LiBH₄) 2 M in hexane (106.28 mmol, 53 mL) was added slowly. The reaction mixture was further stirred 1 h at - 78 °C, and then quenched carefully with saturated solution of NH₄Cl (40 mL) for 0.5 h. (temperature increased from - 78 °C to room temperature). The mixture was partitioned with H₂O (40 mL) and tert-butyl methyl ether (TBME) (90 mL). Water layer was extracted with TBME (25 mL). The organic phases was combined, dried (MgSO₄) and the volatiles were removed in vacuo. The residue was yellow pale oil (62 g).

To the residue, a THF:H₂O 3:1 (80 mL) was added at room temperature under N₂ atmosphere. Then, solid AcONa (53.64 mmol, 4.40 g) was added in one portion and the mixture was cooled to 0 °C. Then H₂O₂ 30% (30 mL, 5 equivalents) was added dropwise during 10 min and the mixture was stirred further 10 min at 0 °C and 30 min at room temperature. Then the mixture was cooled to 0 °C and a saturated solution of Na₂S₂O₃ (30 mL) was added slowly. The reaction mixture was stirred further 10 min at 0 °C and 15 min at room temperature. Then a mixture of H₂O (20 mL) and TBME (35 mL) was added. The organic layer was poured of. Water layer was extracted with TBME (10 mL). The organic phases were combined, dried (MgSO₄) and the volatiles was removed in vacuo. Then the resulting oily substance (49.2 g) was isolated by flash chromatography on silica gel (Hexane-ethyl acetate from 90:10 to 60:40) to give 9.13 g of a mixture of diols enantiomeric ratio (e.r). 80:20 and Sin/Anti ratio 90:10 (yield 66%). Recrystallization in hexanes affords the title compound as a white crystalline solid (5.2 g, 57% overall yield, e.e. 96%). Mp (°C): 80 - 82. Rf (hexane:AcOEt 80:20) = 0.3. [α]_{D} = + 2.3 (c 1.0, CHCl₃). IR (Film) (cm⁻¹): 3349, 2956, 2176. EM (+ESI high resolution): m/z 221.0969 [M+Na]⁺.

### Example 2: Preparation of (3S,5R)-5-(tert-butyldimethylsilyloxy)-7-(trimethylsilyl)hept-1-en-6-yn-3-ol (X)

To a solution of diol (XI) (5.0 g, 25.2 mmol) and imidazole (2.1 g, 30.3 mmol) in anhydrous THF (60 mL) at 0 °C under N₂ atmosphere, a solution of TBSCI (4.23 g, 27.7 mmol) in anhydrous THF (20 mL) was added dropwise and the reaction mixture was warmed up to room temperature. The reaction mixture was stirred for 5 h. Then, a 22% solution of NH₄Cl (25 mL) was added slowly and the reaction mixture was stirred 10 min. The mixture was partitioned and the organic phase was dried (MgSO₄), and the volatiles were removed in vacuo. Then the resulting oily substance was purified by flash chromatography on silica gel (hexane-ethyl acetate from 90:10 to 80:20) to give 4.84 g (61 %) of the compound of the title. Light yellow oil. Rf (hexane:AcOEt 80:20) = 0.55. [α]_{D} = + 39.9 (c 1.0, CHCl₃). IR (Film) (cm⁻¹): 3424, 3081, 2958, 2172.

### Example 3: Preparation of (3S,5R)-5-(tert-butyldimethylsilyloxy)hept-1-en-6-yn-3-ol (IXa)

K₂CO₃ (0.101 g, 0.73 mmol) was added in one portion to a stirred solution of (X) (0.455 g, 1.46 mmol) in anhydrous methanol (MeOH) (4.5 mL) at room temperature under N₂ atmosphere. The reaction mixture was stirred for 1 h. After completion of the reaction, the volatiles was removed and CH₂Cl₂ (10 mL) was added to the residue. The mixture was filtered, dried (MgSO₄) and the volatiles were removed to give 0.366 g (yield: 100%) of compound of the title. Light yellow oil. Rf (hexane:AcOEt 80:20) = 0.43. [α]_{D} = + 32.7 (c 1.0, CHCl₃). IR (Film) (cm⁻¹): 3417, 3079, 2956, 2109.

### Example 4: Preparation of (1S,3R)-3-(tert-butyldimethylsilyloxy)-1-(oxiran-2-yl)pent-4-yn-1-ol (VIII)

A suspension solution of mCPBA 77% (13.64 g, 60.83 mmol) in anhydrous CH₂Cl₂ (30 mL) at room temperature under N₂ atmosphere was added to a stirred solution of (IX) (5.85 g, 24.33 mmol) in anhydrous CH₂Cl₂ (20 mL). The reaction mixture was stirred at room temperature for several hours. After completion of the reaction, the reaction mixture was filtered and washed with saturated Na₂S₂O₃ solution (35 mL), saturated NaHCO₃ (2 × 15 mL). The combined organic phases were dried (MgSO₄) and the volatiles were removed to give the compound of the title (5.53 g, yield: 89%) as yellow oil. ¹H NMR (400 MHz, CDCl₃ δ 4.56 (tt, J = 6.65, 6.65, 2.21, 2.21 Hz, 1 H), 3.86 (td, J = 8.17, 3.98, 3.98 Hz, 1 H), 3.72-3.65 (m, 1 H), 2.95 (ddd, J = 8.03, 4.10, 1.96 Hz, 1 H), 2.71 (td, J = 5.09, 3.45, 3.45 Hz, 1 H), 2.69-2.63 (m, 1 H), 2.38 (dd, J = 7.67, 2.10 Hz, 1 H), 1.98-1.81 (m, 1 H), 0.81 (s, 1 H), 0.08 (s, 1 H), 0.08 (s, 1 H), 0.06 (s, 1 H), 0.05 (s, 1 H). ¹³C NMR (101 MHz, CDCl₃ δ 84.3, 73.4, 73.4, 69.5, 68.0, 61.5, 61.1, 55.0, 54.1, 44.7, 44.2, 42.2, 41.7, 25.6, 18.0, - 4.4, -5.1.

### Example 5: Preparation of (1S,3R)-3-(tert-butyldimethylsilyloxy)-1-(oxiran-2-yl)pent-4-ynyl acetate (VII)

To a solution of (VIII) (5.5 g, 21.45 mmol) and catalytic amount of DMAP in anhydrous CH₂Cl₂ (50 mL) at 0 °C under N₂ atmosphere, NEt₃ (3.8 mL, 27.89 mmol) was added dropwise. Then, Ac₂O (2.4 mL, 25.74 mmol) was added thereto dropwise at 0 - 5 °C. The reaction mixture was warmed to room temperature and stirred for 1 h. Then, a saturated NH₄Cl solution (35 mL) was added slowly and the reaction mixture was stirred 10 min. The mixture was partitioned and the aqueous phase was extracted with CH₂Cl₂ (20 mL). The organic phases were combined and washed with saturated NaHCO₃ solution (30 mL). The basic aqueous phase was extracted with CH₂Cl₂ (20 mL). The organic phases were combined, dried (MgSO₄), and the volatiles were removed in vacuo to afford the compound of the title as a yellow pale oil (6.39 g, yield: 99%). ¹H NMR (400 MHz, *CDCl*₃δ 5.07-4.99 (m, 1 H), 4.54-4.48 (m, 1H), 3.16 (ddd, *J* = 5.71, 4.12, 2.63 Hz, 1 H), 3.05 (ddd, *J* = 4.87, 3.88, 2.70 Hz, 1 H), 2.82 (dd, *J* = 4.81, 4.18 Hz, 1 H), 2.77-2.70 (m, 1 H), 2.67 (dd, *J* = 4.84, 2.63 Hz, 1 H), 2.44 (d, *J* = 2.14 Hz, 1 H), 2.43 (d, *J* = 2.14 Hz, 1 H), 2.08 (s, 1H), 2.06 (s, 1 H), 2.16-1.97 (m, 1 H), 0.90 (s, 1 H), 0.15 (s, 1 H), 0.14 (s, 1 H), 0.12 (s, 1 H), 0.12 (s, 1 H). ¹³C NMR (101 MHz, *CDCl*₃δ169.9, 84.0, 83.9, 73.3, 73.2, 70.9, 69.8, 59.6, 52.9, 52.1, 45.1, 39.8, 39.6, 25.6, 20.9, 20.9, 18.0, -4.5, -5.1.

### Example 6: Preparation of (1S,2R,4R)-4-(tert-butyldimethylsilyloxy)-2-(hydroxymethyl)-3-methylenecyclopentyl acetate (VIa)

Strictly deoxygenated anhydrous THF (15 mL) was added to a mixture of Cp₂TiCl₂ (0.166 g, 0.67 mmol) and activated Zn powder (0.65 g, 10.05 mmol) under N₂ atmosphere and the suspension was stirred at room temperature until it turned lime green. Then, a solution of epoxide (VII) (1.0 g, 3.35 mmol) and dry 2,4,6-collidine (2.6 mL, 20.01 mmol) in strictly deoxygenated anhydrous THF (18.5 mL) was added. Then, TMSCI (1.3 mL, 10.05 mmol) was added slowly. The reaction mixture was stirred for 4 h and filtered through Celite®. The diatomaceous earth was washed with TBME (20 mL). The organic phases were combined, washed with 2 M HCl (2 × 10 mL), H₂O (10 mL) saturated NaHCO₃ solution (5 mL) and dried (MgSO₄). The volatiles was removed and the resulting oily substance (0.98 g) was isolated by flash chromatography on silica gel (Hexane-ethyl acetate from 90:10 to 60:40) to give 0.40 g (41 % yield) of the compound of the title. Light yellow oil. [α]_{D} = - 43.5 (c 1.0, CHCl₃). IR (Film) (cm⁻¹): 3447, 3085, 2955, 1734. Ratio cis:trans 5:95. The subproduct of reductive elimination is in an amount less than 5% w/w.

Similar results were obtained by reproducing Example 6 using 2Me-THF as solvent.

### Example 7: Preparation of (1S,2R,4R)-4-(tert-butyldimethylsilyloxy)-2-(hydroxymethyl)-3-methylenecyclopentyl acetate (VIa)

Strictly deoxygenated anhydrous THF (33.5 mL) was added to a mixture of epoxide (VII) (1 g, 3.35 mmol) and dry 2,4,6-collidine·HCl (1.58 mL, 10.05 mmol).Then, Cp₂TiCl₂ (0.166 g, 0.67 mmol) and Mn powder (0.368 g, 6.7 mmol) was added under N₂ atmosphere and the suspension was stirred at room temperature for 4 h and filtered through Celite®. The diatomaceous earth was washed with TBME (20 mL). The organic phases were combined, washed with 2 M HCl (2 × 10 mL), H₂O (10 mL) saturated NaHCO₃ solution (10 mL) and dried (MgSO₄). Then the resulting oily substance (1.15 g) was isolated by flash chromatography on silica gel (Hexane-ethyl acetate from 90:10 to 60:40) to give 0.42 g (42 % yield) of the compound of the title. Ratio cis:trans 5:95. The subproduct of reductive elimination is in an amount less than 5% w/w.

### Example 8: Preparation of ((1R,3R,5S)-5-acetoxy-3-(tert-butyldimethyl silyloxy -2-methylene cyclopentyl) methyl acetate (V)

Strictly deoxygenated anhydrous THF (43 mL) was added to a mixture of Cp₂TiCl₂ (0.69 g, 2.76 mmol) and activated Zn powder (2.7 g, 41.1 mmol) under N₂ atmosphere and the suspension was stirred at room temperature until it turned lime green. Then, a solution of epoxide (VII) (4.12 g, 13.8 mmol) and dry 2,4,6-collidine (14.6 mL, 110.4 mmol) in strictly deoxygenated anhydrous THF (95 mL) was added. Then, TMSCI (7 mL, 55.2 mmol) was added slowly. The reaction mixture was stirred for 4 h and filtered through Celite®. The diatomaceous earth was washed with TBME (75 mL). The organic phases were combined, washed with 2 M HCl (3 × 30 mL), H₂O (30 mL) saturated NaHCO₃ solution (30 mL), dried (MgSO₄). The volatiles was removed and the residue poured in to hexane (10 mL), MeOH (60 mL) and H₂O (30 mL). The resulting organic layer was separated and extracted thoroughly with MeOH:H₂O system. The aqueous phases were combined and the volatiles were removed. The resulting aqueous phase was extracted with TBME (2 × 30 mL). The combined organic phases were dried (MgSO₄) and the volatiles were removed. Then the resulting oily substance (3.72 g) was dissolved in CH₂Cl₂ (20 mL) at room temperature under N₂ atmosphere. Catalytic amount of DMAP was added to the solution and cooled to 0 °C. Then, NEt₃ (1.58 mL) and Ac₂O (1 mL) was added slowly. The reaction mixture warmed up to room temperature and stirred for 1 h. Then, a saturated NH₄Cl solution (20 mL) was added slowly and the reaction mixture was stirred 10 min. The mixture was partitioned and the aqueous phase was extracted with CH₂Cl₂ (20 mL). The organic phases were combined and washed with saturated NaHCO₃ solution (30 mL). The basic aqueous phase was extracted with CH₂Cl₂ (20 mL). The organic phases were combined, dried (MgSO₄), and the volatiles were removed in vacuo. Then the resulting oily substance (3.89 g) was isolated by flash chromatography on silica gel (Hexane-ethyl acetate from 90:10 to 80:20) to give 1.83 g (44 % yield, 2 steps) of the compound of the title. Light yellow oil. [α]_{D} = - 23.3 (*c* 1.0, CHCl₃). IR (Film) (cm⁻¹): 3095, 2956, 1744.

### Example 9: Preparation of ((1R,3R,5S)-5-acetoxy-3-(tert-butyldimethyl silyloxy -2-methylene cyclopentyl) methyl acetate (V)

To a solution of (VIa) (2.26 g, 7.53 mmol) and catalytic amount of DMAP in anhydrous CH₂Cl₂ (15 mL) at 0 °C under N₂ atmosphere, NEt₃ (1.44 mL, 10.54 mmol) was added dropwise. Then, Ac₂O (0.92 mL, 9.79 mmol) was added thereto dropwise at 0 - 5 °C. The reaction mixture was warmed to room temperature and stirred for 1 h. Then, a saturated NH₄Cl solution (15 mL) was added slowly and the reaction mixture was stirred 10 min. The mixture was partitioned and the aqueous phase was extracted with CH₂Cl₂ (20 mL). The organic phases were combined and washed with saturated NaHCO₃ solution (15 mL). The basic aqueous phase was extracted with CH₂Cl₂ (10 mL). The organic phases were combined, dried (MgSO₄) and the volatiles were removed in vacuo to afford the compound of the title as a yellow pale oil (2.43 g, 94%).

### Example 10 Preparation of (1S,2R,4R)-4-((tert-butyldimethylsilyl)oxy)-2-(hydroxymethyl)-3-methylenecyclopentanol (VIb)

Strictly deoxygenated anhydrous THF (11 mL) was added to a mixture of epoxide (VIII) (0.5 g, 1.95 mmol) and activated Zn powder (0.38 g, 5.85 mmol) under N₂ atmosphere at room temperature. Then, dry 2,4,6-collidine (2.07 mL, 15.6 mmol) was added dropwise. Then, TMSCI (0.99 mL, 7.8 mmol) was added slowly. The reaction mixture was stirred 30 min. Finally, a solution of Cp₂TiCl₂ (97 mg, 0.39 mmol) in Strictly deoxygenated anhydrous THF (7 mL) was added dropwise and the reaction mixture was stirred for 1 h. Water (5 mL) was added and the mixture was stirred for 10 min and filtered through Celite®. The diatomaceous earth was washed with TBME (10 mL). The organic phases were combined and acidified with 2 M HCl until pH = 2. The mixture was stirred 15 min and the phases were separated. The organic phase was washed with H₂O (2 × 5 mL) and dried (Na₂SO₄). The volatiles was removed and the resulting oily substance was 0.49 g. Estimated yield by RMN-¹H was 39 %. Ratio cis:trans 5:95. The subproduct of reductive elimination is in an amount less than 5% w/w.

### Example 11 Preparation of ((1R,3R,5S)-5-acetoxy-3-(tert-butyldimethyl silyloxy -2-methylene cyclopentyl) methyl acetate (V)

To a solution of (1S,2R,4R)-4-((tert-butyldimethylsilyl)oxy)-2-(hydroxymethyl)-3-methylenecyclopentanol (VIb) (0.200 g, 0.78 mmol) and catalytic amount of DMAP in anhydrous CH₂Cl₂ (5 mL) at 0 °C under N₂ atmosphere, NEt₃ (0.27 mL, 1.95 mmol) was added dropwise. Then, Ac₂O (0. 18 mL, 1.88 mmol) was added thereto dropwise at 0 - 5 °C. The reaction mixture was warmed to room temperature and stirred for 1 h.

Then, a saturated NH₄Cl solution (5 mL) was added slowly and the reaction mixture was stirred 10 min. The mixture was partitioned and the aqueous phase was extracted with CH₂Cl₂ (10 mL). The organic phases were combined and washed with saturated NaHCO₃ solution (5 mL). The basic aqueous phase was extracted with CH₂Cl₂ (10 mL). The organic phases were combined, dried (MgSO₄), and the volatiles were removed in vacuo to afford compound (V) as a yellow pale oil (0.246 g, 92%).

### Example 12: Preparation of ((1R,3R,5S)-5-acetoxy-3-hydroxy-2-methylenecyclo-pentyl) methyl acetate (IV)

To a solution of (V) (2.43 g, 7.09 mmol) in anhydrous THF (9 mL) at 0 °C under N₂ atmosphere, a 70% solution of HF in pyridine (3 mL, 28.27 mmol) was added dropwise and the reaction mixture was warmed up to room temperature. The reaction mixture was stirred for 2 h. Then, H₂O (3 mL) was added slowly and the reaction mixture was stirred 10 min. The resulting solution was extracted with AcOEt (3 × 20 mL). The combined organic phase were dried (MgSO₄), and the volatiles were removed in vacuo. Then the resulting oily substance (1.66 g) was isolated by flash chromatography on silica gel (Hexane-ethyl acetate from 60:40 to 40:60) to give 1.37 g (85 % yield) of the compound of the title.

### Example 13: Preparation of ((1 R,3S,5S)-5-acetoxy-3-(2-amino-6-chloro-9H-purin-9-yl)-2-methylenecyclopentyl)methyl acetate (II)

To a solution of (IV) (1.36 g, 5.958 mmol) and triphenylphospine (3.12 g, 11.92 mmol) in anhydrous THF (268 mL) at 0 °C under N₂ atmosphere, a suspension of 2-amino-6-chloroguanine (III) (2.02 g, 11.92 mmol) in anhydrous DMF (30 mL) was added. The mixture was cooled to - 40 °C, and DIAD (2.34 mL, 11.92 mmol) was added dropwise. The reaction mixture was stirring for 1 h. then, the reaction mixture warmed up and poured into TBME (40 mL), washed with 0.1 M NaOH (40 mL), dried (MgSO₄) and the volatiles were removed in vacuo. Purification by flash chromatography on silica gel (Hexane-ethyl acetate from 40:60 to 30:70) gave 1.15 g (51 % yield) of the compound of the title.

### Example 14: Preparation of entecavir

To (II) (0.4 g, 1.05 mmol) at room temperature, a 2.5 M solution of NaOH (10 mL) was added. The reaction mixture was heated to 80 °C for 2 h. The reaction mixture was cooled to 0 °C and neutralized with HCl to pH = 7. Decolorizing carbon (0.5 g) was added and the mixture was heated at 90 °C for 1 h. The hot mixture was filtered through Celite®. The filtrate was cooled under stirring to effect crystallization. The estimated yield was 60 % approximately.

### Comparative Example 15: Comparison of the effect of the base used in the monoprotection reaction of (3S,5R)-5-(tert-butyldimethylsilyloxy)-7-(trimethylsilyl)hept-1-en-6-yn-3-ol

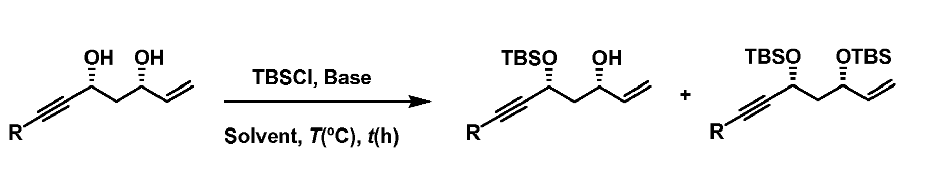

To a stirred solution of the starting material of the previous scheme (0.200 g, 1 mmol),a base and acatalytic amount of DMAP in a solvent (0.5 M), at 0 °C and under N₂ atmosphere, a 0.6 M solution of TBSCI was added dropwise. The reaction mixture was stirred for t (h) at 15 °C. Then, a 22% solution of NH₄Cl (2 mL) was added slowly and the reaction mixture was stirred 10 min. The mixture was partitioned, the organic phase was dried (MgSO₄), and the volatiles were removed in vacuo. The resulting oily substance was purified by flash chromatography on silica gel (Hexane-ethyl acetate from 90:10 to 80:20).

Table 1 below show the conditions and results of several Examples which show the effect of the base in the selectivity of the monoprotection reaction in the propargylic position of the compound of formula (XI).

**Table 1:**

| Example | R | Eq. TBS | Base (eq.) | DMAP (%) | Solvent | T (°C) | T (h) | % mono | % di |
|---|---|---|---|---|---|---|---|---|---|
| Example 15a | TMS | 1.1 | Imidazole (1.2) | 0 | THF | 15 | 6 | 67 | 11 |
| Comparative Example 15b | TMS | 1.1 + 0.5 | Pyr | 10 | Pyr | 15 | 96 | < 40 | 30 |
| Comparative Example 15c | TMS | 1.1 | DIPEA | 10-15 | DCM | 15 | 15 | 35 (mixture) | 30 |

### Comparative Example 16: Comparison of the effect of the protective groups used in the radical cyclization

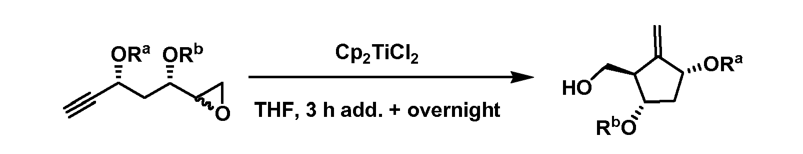

General process:
Generation of Cp₂TiCl:
   To a solution of titanocene dichloride (0.400 g, 1.61 mmol) in deoxigenated freshly distilled THF (20 mL) under N₂ was added activated zinc dust (0.320 g, 4.84 mmol). The resultant dark red solution was stirred for 1 h, during which time the heterogeneous solution turned a deep lime green. Stirring was discontinued and the residual zinc was allowed to settle. The resulting green solution of Cp₂TiCl was 0.08 M.
Reaction:
   A 0.08 M solution of Cp₂TiCl (17 mL, 1.36 mmol) (vide supra) was added dropwise via syringe pump over 3 h to a stirred solution of the corresponding starting epoxide (see reaction scheme where R^{a} and R^{b} are as in Table 2) (0.46 mmol) dissolved in deoxigenated freshly distilled THF (23 mL) at room temperature under an N₂ atmosphere. The reaction mixture was stirred for 8 h.
Treatment A:
   A 10% aq. H₂SO₄ (10 mL) was added and stirred for 5 min. Then, the solution was extracted thoroughly with EtOAc. The bright orange aqueous portion was extracted further with EtOAc. The combined organic phases were washed with saturated aq. NaHCO₃ (30 mL) and dried (MgSO₄) and concentrated in vacuo. The crude residue was purified by flash chromatography (5% EtOAc/hexane).
Treatment B:
   A saturated solution of NH₄Cl (10 mL) was added and the resulting mixture was stirred for 2 h. Then, the solution was extracted thoroughly with EtOAc. The bright orange aqueous portion was extracted further with EtOAc. The combined organic phases were washed with saturated aq. NaHCO₃ (15-20 mL) and dried (MgSO₄) and concentrated in vacuo The crude residue was purified by flash chromatography.

The results obtained are summarized in Table 2.

**Table 2:**

| Examples | R^{a} | R^{b} | Yield (%) | trans:cis | Treatment carried out once completed the reaction |
|---|---|---|---|---|---|
| Comparative Example 16a Reproduction of Example 3.1.11 of Ziegler et al Tetrahedron 2003 vol. 59, pp 9013-9018 | TBS | TBS | 30 | 95:5 | Treatment A |
| Comparative Example 16b | TBS | TBS | 50 | 95:5 | Treatment B |
| Comparative Example 16c | TBS | Ac | 40 | 95:5 | Treatment B |
| Comparative Example 16d | TBDPS | TBDPS | 8 | > 97:3 | Treatment B |
| Comparative Example 16e | TIPS | TIPS | 0 | - | Treatment B |
| Comparative Example 16f | TIPS | OH | 0 | - | Treatment B |

The comparison between protective groups is given for the process carried out with stoichiometric amounts of catalyst. The results show that the cyclization can be carried out with hydroxyls differentiated provided that there is a TBS group in the propargylic position. Besides, the yield obtained in comparative Example 16c carried out with a stoichiometric amount of catalyst is of the same order than the yields obtained in Example 6 carried out with a catalytic amount of catalyst. However, in the Examples 16a-16d it was found the product of reductive elimination is obtained in an amount comprised between 10 and 20%. These results illustrate that the catalytic radicalary cyclization with TBS ether and an ester as protective groups proceeds with similar yields than the stoichiometric radicalary cyclization and has the advantage that is industrializable.

### REFERENCES CITED IN THE APPLICATION

- EP481754-A
- CN1566118-A
- Li Wei Guo et al., "A new route for synthesis of Entecavir", Chinese Chemical Letters 2006 vol. 17, pp.907-910
- CN1861602-A
- W02004/52310-A
- W02010/74534-A
- F.E: Ziegler et al., "Radical cyclization studies directed toward the synthesis of BMS-200475 'entecavir': the carbocyclic core", Tetrahedron 2003, vol. 59, pp 9013-9018)
- J.A. Funel et al., "Enyne versus diene RCM in the synthesis of cyclopentene derivatives toward the A ring of FR181877", J.Org. Chem. 2004 vol. 69, pp. 4555-4558
- Peter Mohr, "Stereoselective synthesis of syn-1,3-diols", Tetrahedron letters 1991, vol. 32, pp. 2219-2229.

## Claims

1. A process for the preparation of entecavir of formula (I), or a pharmaceutically acceptable salt thereof, 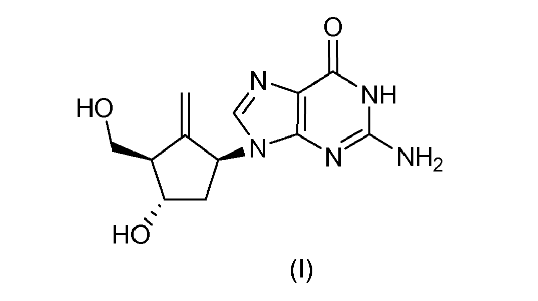 which comprises submitting a compound of formula (VIII) to a double esterification and to a catalytic radicalary cyclization using titanocene dichloride as catalyst in the presence of Mn/2,4,6-collidine HCl or Zn/2,4,6-collidine/trimethylsilyl chloride, yielding to a compound of formula (V), 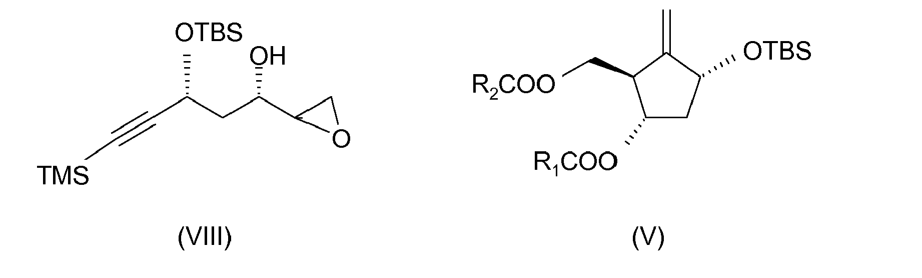 wherein:
either a compound of formula (VIII) is submitted to a first esterification reaction, yielding to a compound of formula (VII); 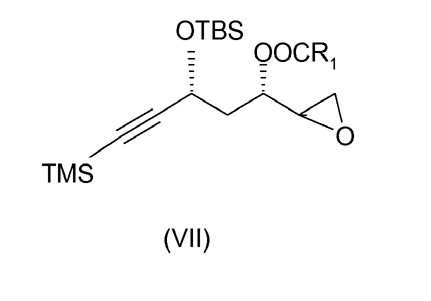 then to the catalytic radicalary cyclization to yield a compound of formula (VIa); followed by a second esterification reaction, to yield a compound of formula (V); 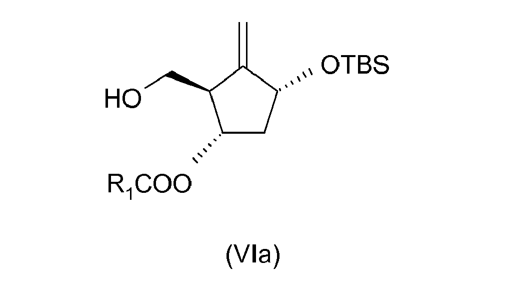 or, alternatively,
the compound of formula (VIII) is submitted first to the catalytic radicalary cyclization, to yield a compound of formula (VIb); and then to a esterification reaction, to yield a compound of formula (V); and 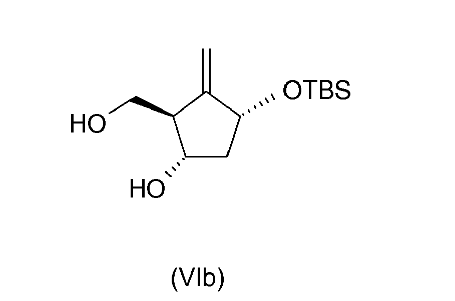 wherein:
R₁ and R₂ are radicals independently selected from the group consisting of (C₁-C₆)-alkyl, phenyl, and phenyl substituted by a radical selected from the group consisting of (C₁-C₄)-alkyl, (C₁-C₄)-alkoxy, halogen, and nitro.

2. The process according to claim 1, wherein the esterification reaction is an acetylation reaction.

3. The process according to any of the claims 1-2, wherein the compound of formula (VIII) is transformed into the compound of formula (V) by the sequence of reactions comprising a first esterification, a catalytic radicalary cyclization, and a second esterification.

4. The process according to any of the claims 1-3, wherein the amount of titanocene chloride is comprised between 15-25% w/w in respect of compound of formula (VII) or of compound of formula (VIII).

5. The process according to any of the claims 1-4, wherein the solvent of the radical cyclization is tetrahydrofuran or 2-methyl tetrahydrofuran.

6. The process according to any of the claims 1-5, further comprising submitting a compound of formula (X) to a epoxidation reaction and to a desilylation reaction, 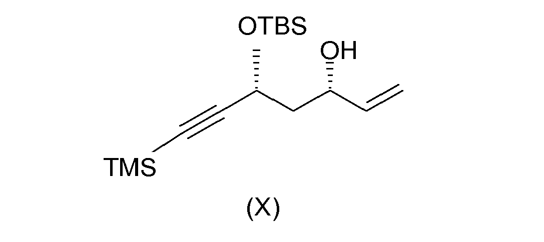 wherein
either a compound of formula (X) is first submitted to a desilylation reaction to yield a compound of formula (IXa) and then to a epoxidation reaction using an epoxidating agent in the presence of a suitable solvent to yield the compound of formula (VIII); 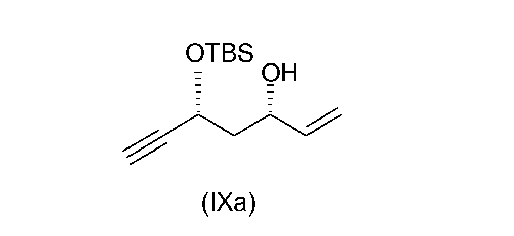 or, alternatively, a compound of formula (X) is first submitted to an epoxidation reaction using an epoxidating agent in the presence of a suitable solvent to yield a compound of formula (IXb) and then to a desilylation reaction to yield the compound of formula (VIII).

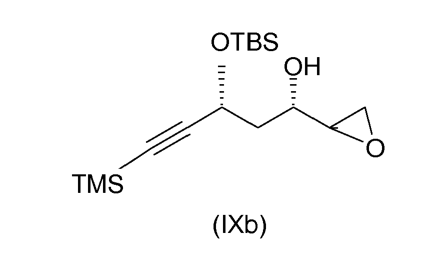

7. The process according to claim 6, further comprising a previous step wherein a compound of formula (XI) is reacted with tert-butyldimethylsilyl chloride, in the presence of imidazole and a suitable solvent to yield a compound of formula (X).

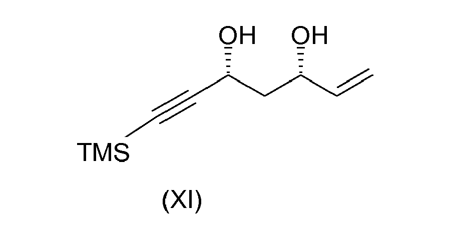

8. The process according to claim 7, further comprising a previous step wherein a compound of formula (XII) is reacted with (+)-B-chlorodiisopinocampheylborane in the presence of an organic tertiary amine and a suitable solvent and then with a compound of formula (XIII), followed by submitting the compound thus obtained to a syn stereoselective reduction using a reducing agent, and then to a treatment to cleavage the boron moiety.

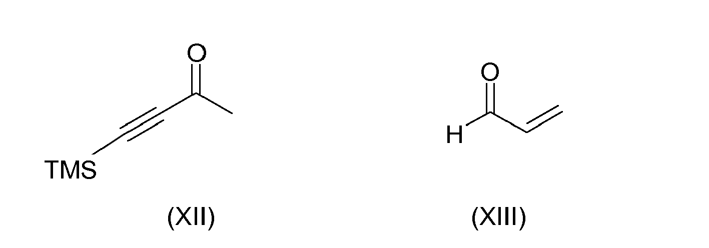

9. The process according to any of the claims 1-8, further comprising first submitting the compound of formula (V) to a desilylation reaction to remove the TBS group with a desilylating agent in the presence of a suitable solvent, yielding to a compound of formula (IV), 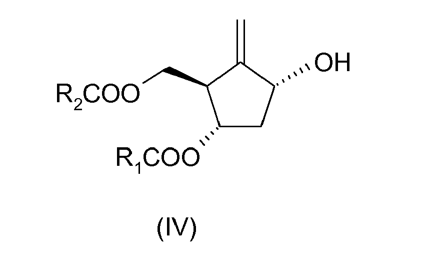 then, carrying out a Mitsunobu reaction between the compound of formula (IV) and a compound of formula (III) to yield a compound of formula (II), 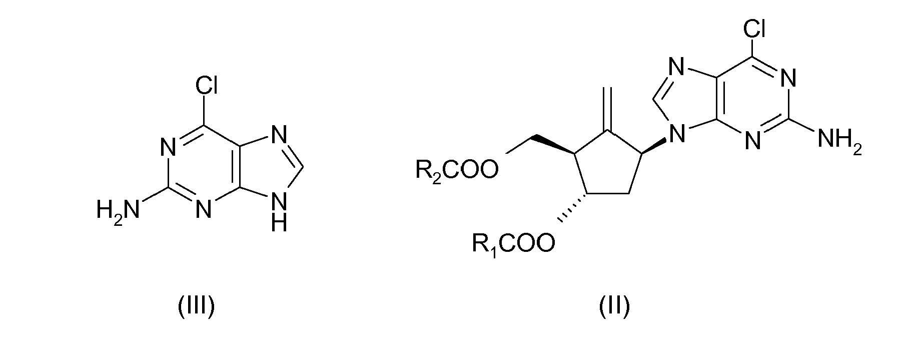 followed by submitting the compound of formula (II) to a hydrolysis reaction yielding entecavir, and if desired reacting the entecavir obtained with a pharmaceutically acceptable acid or with a pharmaceutically acceptable base to yield the corresponding pharmaceutically acceptable salt;
wherein:
R₁ and R₂ are radicals independently selected from the group consisting of (C₁-C₆)-alkyl, phenyl, and phenyl substituted by a radical selected from the group consisting of (C₁-C₄)-alkyl, (C₁-C₄)-alkoxy, halogen, and nitro.

10. A compound of formula (VIa) wherein R₁ is a radical selected from the group consisting of (C₁-C₆)-alkyl, phenyl, and phenyl substituted by a radical selected from the group consisting of (C₁-C₄)-alkyl, (C₁-C₄)-alkoxy, halogen, and nitro.

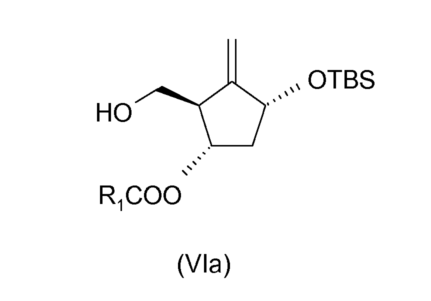

11. A compound of formula (VII) wherein R₁ is a radical selected from the group consisting of (C₁-C₆)-alkyl, phenyl, and phenyl substituted by a radical selected from the group consisting of (C₁-C₄)-alkyl, (C₁-C₄)-alkoxy, halogen, and nitro.

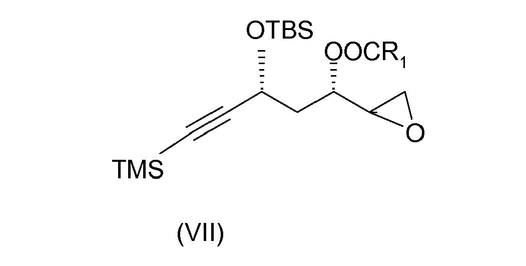

12. A compound of formula (VIII).

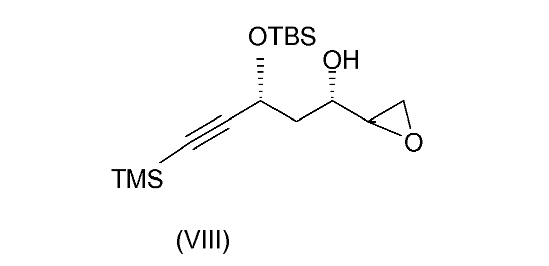

13. A compound of formula (IX).

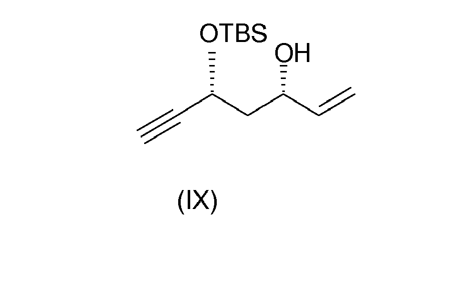

14. A compound of formula (X).

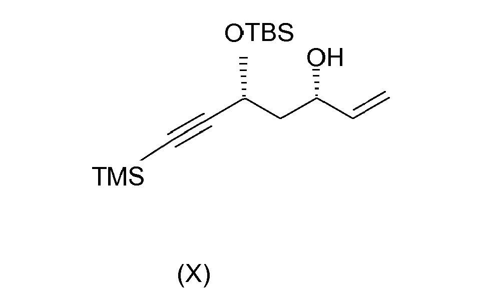

15. A compound of formula (XI).
